(19) Europäisches Patentamt
European Patent Office
Office européen des brevets

(11) **EP 3 313 265 B1**

(12) **EUROPÄISCHE PATENTSCHRIFT**

(45) Veröffentlichungstag und Bekanntmachung des Hinweises auf die Patenterteilung:
**30.03.2022   Patentblatt 2022/13**

(21) Anmeldenummer: **16733929.0**

(22) Anmeldetag: **28.06.2016**

(51) Internationale Patentklassifikation (IPC):
***A61B 3/107*** (2006.01)

(52) Gemeinsame Patentklassifikation (CPC):
**A61B 3/107; A61B 3/14**

(86) Internationale Anmeldenummer:
**PCT/EP2016/064954**

(87) Internationale Veröffentlichungsnummer:
**WO 2017/001375 (05.01.2017 Gazette 2017/01)**

(54) **VERFAHREN UND VORRICHTUNG ZUR BESTIMMUNG DER POSITION UND DER LAGE EINES AUGES**

METHOD AND APPARATUS FOR DETERMINING THE POSITION AND THE ORIENTATION OF AN EYE

PROCÉDÉ ET DISPOSITIF DE DÉTERMINATION DE LA POSITION ET DE L'ORIENTATION D'UN OEIL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.06.2015   DE 102015008217**

(43) Veröffentlichungstag der Anmeldung:
**02.05.2018   Patentblatt 2018/18**

(73) Patentinhaber: **Precitec Optronik GmbH
63263 Neu-Isenburg (DE)**

(72) Erfinder:
• **PALZER, Gabriel
63110 Rodgau (DE)**

• **DIETZ, Christoph
63179 Obertshausen (DE)**
• **BIRMANNS, Stefan
8462 Rheinau (CH)**

(74) Vertreter: **Ostertag & Partner Patentanwälte mbB
Azenbergstraße 35
70174 Stuttgart (DE)**

(56) Entgegenhaltungen:
WO-A1-2015/011692      DE-A1-102004 052 199
DE-A1-102010 024 407

**Beschreibung**

Die Erfindung betrifft ein Verfahren und eine Vorrichtung zur Bestimmung der Position und der Lage eines Auges.

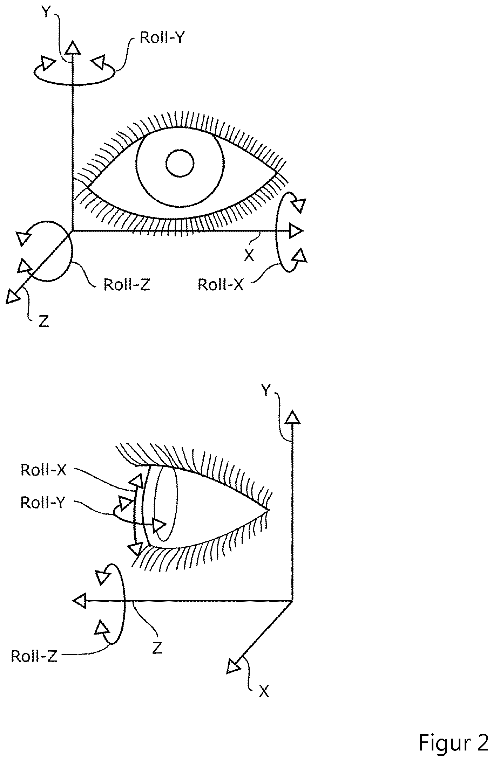

Derartige Verfahren und Vorrichtungen werden insbesondere bei der refraktiven Chirurgie des Auges eingesetzt, bei der die Gesamtbrechkraft des Auges verändert und danach idealerweise konventionelle optische Korrekturen wie Brillen oder Kontaktlinsen entfallen können. Insbesondere bei der Laserablation zur Änderung der Hornhautkrümmung, bei der in der Regel ein Ablationslaserstrahl über die gekrümmte Korneaoberfläche geführt wird, ist es während der Behandlung notwendig, die exakte Position des Auges sowie die Lage des Auges - also die Orientierung des Auges hinsichtlich dreier Winkel im Raum - zu kennen. Die Position des Augapfels n einem kartesischen Koordinatensystem mit den Koordinaten X, Y und Z sowie die Lage des Augapfels bezüglich dreier Raumwinkel $Roll_x$, $Roll_y$ und $Roll_z$ sind gemäß Figur 2 wie folgt definiert:

Die kartesischen Koordinaten X, Y und Z sind so gewählt, dass die Z-Achse im Wesentlichen mit einer Laserbearbeitungsachse A3 zusammenfällt und in Blickrichtung orientiert ist. Die X-Achse ist so orientiert, dass bei einer aufrecht stehenden Person die X-Achse mit der Horizontalen zusammenfällt. Entsprechend ist die Y-Achse vertikal angeordnet. Die Raum- bzw. Drehwinkel des Augapfels sind entsprechend den kartesischen Koordinaten als Rollwinkel $Roll_x$, $Roll_y$ und $Roll_z$ definiert. Der Winkel $Roll_z$ wird auch Zyklotorsion genannt.

Eine Möglichkeit zur Erfassung von Position und Lage des Auges besteht in der Aufnahme eines Kamerabildes der Oberfläche des Auges. Die Auswertung des Kamerabilds muss während einer solchen Operation alle möglichen Bewegungen des Auges berücksichtigen.

Das Dokument DE 10 2010 024 407 A1 beschreibt ein Verfahren und eine Vorrichtung zur Bestimmung der Augenposition. Es wird mittels einer Lichtquelle auf der Oberfläche eines Auges ein Lichtmuster erzeugt und mit einer Bilderfassungseinrichtung erfasst. Dabei werden vier ausgezeichnete Punkte auf der Sklera nahe der Iris ermittelt, welche sich durch Unstetigkeiten oder Unterbrechungen zweier Linien des Lichtmusters ergeben, bedingt durch den Höhenunterschied zwischen Iris und Limbus. Mittels einer Bildverarbeitungseinrichtung wird aus der Position der Punkte die jeweilige Lage der Irisebene und damit auch die Blickrichtung errechnet.Der Erfindung liegt die Aufgabe zugrunde, ein Verfahren und eine Vorrichtung anzugeben, welche eine Ermittlung von Größen zur Bestimmung der Position und der Lage der Kornea eines Auges mit einer ausreichenden Genauigkeit zur Durchführung einer lasergestützten Korneabehandlung ermöglichen.

Die Aufgabe wird durch ein Verfahren gemäß dem unabhängigen Anspruch 1 gelöst. Weitere Ausgestaltungen der Erfindung sind in den Unteransprüchen angegeben. Außerdem wird die Aufgabe durch eine Vorrichtung gemäß dem unabhängigen Anspruch 13 gelöst. Weitere Ausgestaltungen der Vorrichtung sind in den Unteransprüchen angegeben.

Das erfindungsgemäße Verfahren weist die folgenden Schritte auf: Es werden linienförmige Strukturen auf die Iris des Auges, beispielsweise durch Projektion, erzeugt. Mittels einer Kamera wird ein erstes Bild des Auges mit den darauf befindlichen linienförmigen Strukturen aufgenommen. Die Kamera ist dabei bevorzugt mit ihrer Kameraachse auf das Auge ausgerichtet. Unter der Kameraachse wird hier und im Folgenden die optische Achse der Kamera verstanden. Es werden mittels des ersten Bildes ein Abstand, also eine Z-Koordinate, und die Lage der linienförmigen Strukturen relativ zu der Kamera charakterisierende Parameter wie beispielsweise $Roll_x$ und $Roll_y$ bestimmt. Aus diesen Angaben kann dann auf den Abstand und die entsprechenden Parameter für eine Lage der Oberfläche, auf welche die Strukturen projiziert sind, geschlossen werden - also beispielsweise die Iris. Hieraus wiederum können der Abstand und die entsprechenden Parameter für die Lage der Kornea bestimmt werden. Dabei müssen die Parameter für eine Charakterisierung der Lage der linienförmigen Strukturen die Lage nicht vollständig charakterisieren.

Unter dem Begriff linienförmige Strukturen werden beispielsweise Geraden, Geradenabschnitte, Streifen, Kreisbögen, Kreise, Ellipsen, etc. verstanden - also Strukturen, die sich durch Linien darstellen lassen. Die Linien müssen nicht notwendigerweise Geraden sein, sondern können gekrümmt oder auch geschlossen wie bei Ellipsen oder Kreisen sein. Bevorzugt befinden sich die linienförmigen Strukturen auf der Iris. Bei der Bestimmung von Parametern für die Position und die Lage der linienförmigen Strukturen kann beispielsweise die Mitte der linienförmigen Strukturen oder die Kante zwischen Hell und Dunkel verwendet werden. Unter der Lage der linienförmigen Strukturen relativ zu der Kamera ist eine winkelabhängige Orientierung der linienförmigen Strukturen im Raum zu verstehen. Die Lage kann beispielsweise durch die Angabe zweier Winkelgrößen definiert sein. Aus der Lage der linienförmigen Strukturen kann auf die Lage des Auges im Raum geschlossen werden. Insbesondere können aus der Lage der linienförmigen Strukturen die zur vollständigen Angabe der Lage des Auges benötigte Winkelgrößen ermittelt werden. Unter der lateralen Position wird die Position des Auges senkrecht zu der Kameraachse oder einer Laserachse verstanden.

Das erfindungsgemäße Verfahren nimmt zur Ermittlung von Größen, die beispielsweise für die Durchführung einer Laserablation der Kornea notwendig sind, nämlich die Lage des Auges und dessen Z-Position, nicht die eigentliche Kornea, sondern die Iris des Auges als Referenz. Auf der Iris werden linienförmige Strukturen projiziert und somit Merkmale erzeugt, die mittels einer Bildverarbeitung einfacher als natürliche Strukturen zu detektieren sind. Die so erzeugten Strukturen bilden einen deutlichen und damit besser zu detektierenden Kontrast, der eine Bildverarbeitung vereinfacht und aus der Bildverarbeitung präzisere Ergebnisse liefert. Gleichzeitig sind die linienförmigen Strukturen aufgrund ihrer weniger variierenden und bekannten Geometrie einfacher in der Bildverarbeitung zu handhaben als natürliche Strukturen.

Bei einer Weiterbildung der Erfindung kann vorgesehen sein, dass mittels einer Kamera ein zweites Bild des Auges ohne darauf befindliche linienförmige Strukturen aufgenommen wird und mittels des zweiten Bildes eine laterale Position, also die Parameter X und Y, und/oder eine Zyklotorsion des Auges, also $Roll_z$, bestimmt werden. Alternativ oder zusätzlich kann mittels eines dritten Bildes die Zyklotorsion bestimmt werden. Die Zyklotorsion gibt eine Verdrehung des Auges um die Kameraachse oder eine Laserachse an. Das Aufnehmen des zweiten und/oder des dritten Bildes kann mit derselben Kamera vorgenommen werden, mit der das erste Bild aufgenommen wird. Mit diesen ergänzenden Schritten wird auf einfache Weise eine vollständige Bestimmung der Position und der Lage eines Auges ermöglicht. Das zweite und/oder dritte Bild kann zu geeigneten Zeitpunkten beispielsweise im Rahmen eines Messzyklus aufgenommen werden. Ein solcher Messzyklus kann beispielsweise eine erstmalige Aufnahme eines ersten Bildes mit linienförmigen Strukturen, eine N-malige Aufnahme weiterer zweiter Bilder ohne linienförmige Strukturen und danach eine Aufnahme eines dritten Bildes vorsehen. Das dritte Bild kann beispielsweise mit einer höheren Auflösung als die zweiten Bilder aufgenommen werden. Aus dem ersten Bild können die Werte für Z und $Roll_x$ und $Roll_y$ bestimmt werden. Aus den zweiten Bildern können neue Werte für X und Y und damit auch für $Roll_x$ und $Roll_y$ bestimmt werden. Aus dem dritten Bild kann $Roll_z$ bestimmt werden.

Eine bevorzugte Ausführungsform sieht vor, dass das Bestimmen der Lage der linienförmigen Strukturen das Bestimmen eines ersten Verkippungswinkels und eines zweiten Verkippungswinkels der linienförmigen Strukturen, insbesondere um Achsen senkrecht zu der Kameraachse, umfasst. Aufgrund der projizierten linienförmigen Strukturen können ein erster und ein zweiter Verkippungswinkel besonders zuverlässig bestimmt werden. Als Bezugsgröße für die Verkippungswinkel kann die Bild- oder Kameraachse dienen. Anhand der Verkippungswinkel der linienförmigen Strukturen kann auf die Lage der Iris und damit auch auf die Lage des Auges geschlossen werden.

Bei einer Ausführungsform des Verfahrens können zur Bestimmung des Abstands und der Lage Parameter ermittelt werden, welche die linienförmigen Strukturen charakterisieren. Bei den Parametern kann es sich beispielsweise um die Position und den Winkel der linienförmigen Strukturen handeln, wenn die linienförmigen Strukturen Geraden oder Streifen darstellen. Bei kreisförmigen oder kreisbogenförmigen Strukturen könnte es sich beispielsweise um eine Position des Mittelpunkts und um einen Radius handeln.

Bevorzugt umfasst das Bestimmen des Abstands und der Lage der linienförmigen Strukturen ein Anwenden einer Houghtransformation. Eine Houghtransformation ist bei linienförmigen Strukturen von Vorteil, die durch Geraden oder Kreise beschreibbar sind. Mittels der Houghtransformation kann besonders vorteilhaft aus einer Vielzahl ermittelter linienförmiger Strukturen die den tatsächlich vorhandenen Strukturen am nächsten kommenden ausgewählt werden.

Alternativ kann bei dem erfindungsgemäßen Verfahren vorgesehen sein, dass das Bestimmen des Abstands und der Lage der linienförmigen Strukturen ein Anwenden einer eindimensionalen Fourier-Transformation umfasst. Insbesondere können mittels der eindimensionalen Fourier-Transformation ermittelte charakterisierende Parameter eine Periode entlang einer Richtung sowie eine Phase umfassen. Das Verfahren der Fourier-Transformation ist ökonomisch in der Anwendung und liefert zuverlässige Ergebnisse.

Vorteilhafterweise umfasst das Bestimmen des Abstands und der Lage das Bestimmen, in dem ersten Bild, einer Position der Gesamtheit der linienförmigen Strukturen, eines Abstands der linienförmigen Strukturen voneinander und/oder eines Winkels der Gesamtheit der linienförmigen Strukturen. Unter der Position der Gesamtheit der linienförmigen Strukturen wird ein geometrischer Schwerpunkt aller erfassten linienförmigen Strukturen oder die Position einer bestimmten linienförmigen Struktur verstanden, aus welcher sich die Position der restlichen linienförmigen Strukturen ableiten lässt. Aus der Position der Gesamtheit der linienförmigen Strukturen in dem ersten Bild lassen sich Rückschlüsse über den Abstand der linienförmigen Strukturen von der Kamera und damit auch über den Abstand der Iris und schlussendlich über den Abstand der Kornea von der Kamera ziehen. Aus dem Abstand einzelner linienförmige Strukturen voneinander oder aus einem mittleren Abstand aller linienförmigen Strukturen kann auf eine Verkippung der linienförmigen Strukturen bezüglich der Kamera geschlossen werden. Gleiches gilt für eine Veränderung der Winkel einzelner linienförmiger Strukturen oder des Winkels der Gesamtheit der linienförmigen Strukturen. Ähnliche Schlussfolgerungen ließen sich aus einer Position der Mittelpunkte sowie einer Elliptizität beziehungsweise aus einer Veränderung derselben ziehen.

Eine vorteilhafte Ausführungsform sieht vor, dass das Bestimmen des Abstands und der Lage ein Einfügen geometrischer Figuren anhand der ermittelten Parameter in das erste Bild umfasst. Grundlage für die geometrischen Figuren können die ermittelten Parameter sein, die sich auf die ursprünglichen linienförmigen Strukturen beziehen. Vorteilhafterweise werden vordem Einfügen die ermittelten Parameter verändert. Bei der Veränderung der Parameter kann es sich beispielsweise um ein Verschieben der die ursprünglichen linienförmigen Strukturen repräsentierenden geometrischen Figuren, um eine Veränderung der Winkel einzelner oder aller geometrischer Figuren zueinander oder/und um ein Verzerren der geometrischen Figuren handeln. Während die linienförmigen Strukturen in dem von der Kamera aufgenommenen ersten Bild durch einzelne Pixel repräsentiert sind, kann durch die Veränderung der ermittelten Parameter vor dem Einfügen der geometrischen Figuren beispielsweise ein Verschieben in einem Subpixel-Maßstab durchgeführt werden. Nach dem nachträglichen Einfügen der ermittelten geometrischen Figuren kann das Bestimmen beispielsweise des Abstands und der Lage der linienförmigen Strukturen erneut durchgeführt werden. In diesem iterativen Prozess kann je nach Qualität, Auflösung und/oder Kontrast des ersten Bildes eine deutliche Verbesserung des Bestimmungsergebnisses erzielt werden.

Bei einer Ausführungsform kann der Schritt des Bestimmens der Zyklotorsion und/oder der Position des Auges ein Aufnehmen eines Referenzbilds umfassen. Anhand des Referenzbilds kann durch Vergleichen mit dem momentan aufgenommenen zweiten Bild eine Verdrehung des Auges um die Kamera- bzw. Laserachse und eine Veränderung der Position des Auges, beispielsweise des Pupillenmittelpunkts, erfasst werden.

Eine Ausführungsform sieht ein Ausgeben der Position oder/und der Lage der Kornea des Auges vor. Die Ausgabe bildet die Schnittstelle zu einer Steuereinrichtung, welche die Position beispielsweise eines Lasers für die Ablation der Kornea festlegt.

Die erfindungsgemäße Vorrichtung zur Bestimmung von Größen für die Position und die Lage der Kornea eines Auges umfasst einen Projektor zur Erzeugung linienförmiger Strukturen auf der Iris des Auges, eine Kamera, die mit ihrer Kameraachse auf das Auge ausgerichtet ist, so wie eine mit dem Projektor und der Kamera verbundene Steuereinrichtung. Die Steuereinrichtung ist dazu eingerichtet, mittels der Kamera ein erstes Bild des Auges mit den linienförmigen Strukturen aufzunehmen und anhand des ersten Bildes einen Abstand und eine Lage der linienförmigen Strukturen relativ zu der Kamera zu bestimmen. Mittels dieser Vorrichtung sind die Vorteile des bereits beschriebenen Verfahrens erzielbar.

Bei einer vorteilhaften Weiterbildung kann die Vorrichtung dazu eingerichtet sein, mittels der Kamera ein zweites Bild des Auges ohne linienförmige Strukturen aufzunehmen und anhand des zweiten Bildes eine Zyklotorsion des Auges und eine laterale Position des Auges zu bestimmen.

Bei einer vorteilhaften Ausgestaltung kann die Steuereinrichtung dazu eingerichtet sein, für die Aufnahme des zweiten Bildes die zweite Zeile der Kamera auszulesen oder/und zwei Pixel zu einem Pixel zusammenzufassen. Somit kann die gleiche Kamera für zwei unterschiedliche Betriebsarten verwendet werden, um höhere Bildwiederholraten und/oder geringere Latenzen zu erreichen.

Des Weiteren kann es von Vorteil sein, wenn eine Beleuchtungseinrichtung vorgesehen ist, die dazu eingerichtet ist, für die Aufnahme des zweiten Bildes die Kornea zumindest teilweise zu beleuchten. Insbesondere zur Erhöhung des Kontrasts bei der Aufnahme des zweiten Bildes kann es von Vorteil sein, die Iris und ihre Umgebung zu erhellen und das Auge flächig auszuleuchten.

Die Erfindung wird nun unter Bezugnahme auf die Zeichnungen näher erläutert. Es zeigen:

Figur 1 ein schematisches Diagramm mit einer Vorrichtung zur Laserbehandlung der Kornea eines Auges;

Figur 2 in einem schematischen Diagramm Koordinaten- und Winkeldefinitionen;

Figur 3 ein Bild der Kamera der Vorrichtung der Figur 1 mit projizierten linienförmigen Strukturen;

Figur 4 eine schematische Darstellung einer Houghtransformation;

Figur 5 eine Maske zur Auswertung des Bildes der Figur 3;

Figur 6 eine schematische Darstellung einer Fourier-Transformation; und

Figur 7 eine Veranschaulichung einer möglichen Korrektur der aus dem Bild gewonnen Daten.

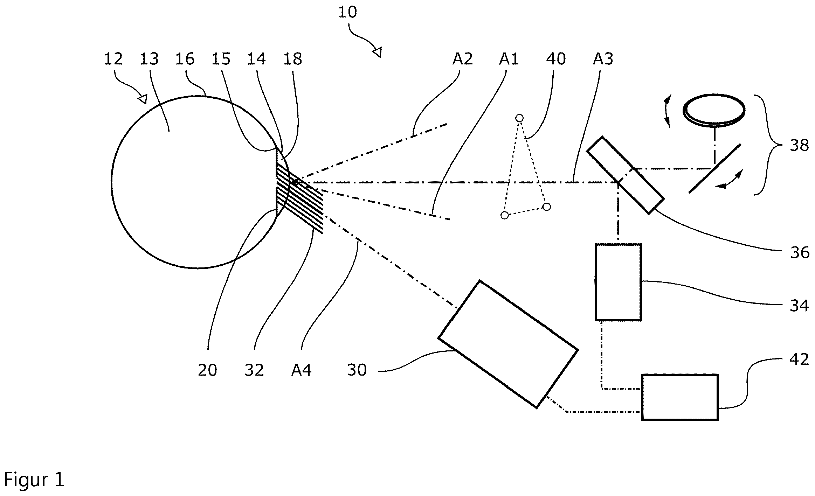

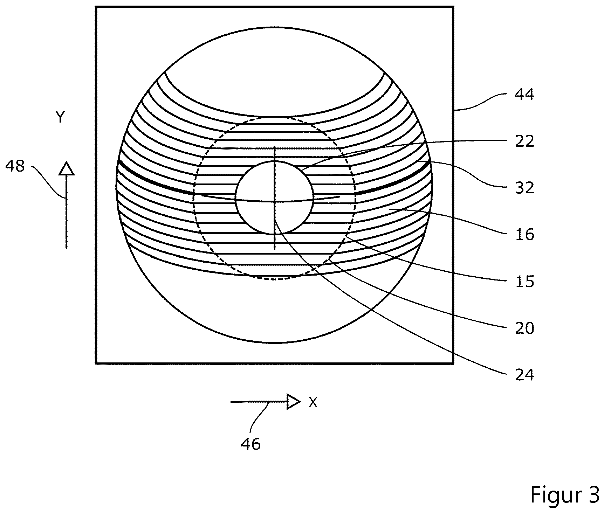

Figur 1 zeigt schematisch den Aufbau eines Systems 10 zur Laserbehandlung eines Auges 12 eines Patienten. Von dem Auge 12 sind in Figur 1 der Augapfel 13, auch Bulbus genannt, mit der äußeren Hornhaut 14, Kornea genannt, gezeigt, an die sich in Umfangsrichtung der Kornea 14 die Lederhaut 16, Sklera genannt, anschließt. In radialer Richtung weiter innen hinter der Kornea 14 befindet sich die vordere Augenkammer 18, die sich von der Kornea 14 bis zur Iris 20 erstreckt. Die Iris 20 trennt die vordere Augenkammer 18 von der hinteren Augenkammer. Der Grenzbereich zwischen Kornea 14 und Sklera 16 wird Limbus 15 genannt. Der äußere Umfang der Iris 20 befindet sich am Limbus 15. Der innere Umfang der Iris 20 bildet die Pupille 22. Dies ist detaillierter in Figur 3 dargestellt.

Die Vorrichtung 10 umfasst einen Projektor 30 zur Projektion eines Streifenmusters 32 auf der Iris 20 des Auges 12, eine Kamera 34, die über einen Strahlteiler 36 frontal auf die Iris 20 des Auges 12 ausgerichtet ist sowie einen Laserstrahlscanner 38 mit einer Galvo-Spiegel-Anordnung, über den eine zur Ablation der Kornea 14 geeigneter Laserstrahl über die Kornea 14 geführt werden kann. Es ist es vorgesehen, über zwei verschiedene Projektionsachsen A1, A2 eine Linie bzw. ein Fadenkreuz auf die Kornea 14 zu projizieren. Ein solches Fadenkreuz 24 ist in Figur 3 dargestellt. Des Weiteren ist eine Beleuchtung mittels dreier Leuchtpunkte 40 vorgesehen. Die Leuchtpunkte beleuchten die Kornea 14 oder die Iris 20 und ermöglichen eine Kontrastverbesserung bei der Aufnahme von Kamerabildern. Neben den genannten Bestandteilen weist das System eine Halterung zur Positionierung des Kopfes des Patienten, einen Fixationslaser als Hilfsmittel zur Positionierung des Kopfes und der Kornea auf, die in Figur 1 nicht dargestellt sind. Figur 1 zeigt außerdem eine Steuereinrichtung 42, die zur Ansteuerung und zur Aufnahme, Verarbeitung und Weitergabe von Daten zumindest der Kamera 34 und des Projektors 30 ausgelegt ist.

Die Position des Augapfels 13 in einem kartesischen Koordinatensystem mit den Koordinaten X, Y und Z sowie die Lage des Augapfels 13 bezüglich dreier Raumwinkel $Roll_x$, $Roll_y$ und $Roll_z$ sind gemäß Figur 2 wie bereits oben beschrieben definiert.

Vor dem Einsatz des Systems 10 sind einige vorbereitende Tätigkeiten durchzuführen. Es ist eine Kalibrierung von Kamera 34 und Projektor 30 zueinander vorzunehmen, d.h. es wird eine Relativpositionierung von Kamera 34 und Projektor 30 ermittelt. Dazu wird anstelle des Auges 12 ein Kalibrierkörper positioniert und das Streifenmuster 32 auf den Kalibrierkörper projiziert. Der Kalibrierkörper kann beispielsweise eine 25 mm mal 45 mm große Fläche mit einer 3 mm hohen Stufe in der Mitte sein und eine weiße und ebene lichtstreuende Oberfläche besitzen. Ein solcher Kalibrierkörper reproduziert die optischen Eigenschaften des Auges 12, insbesondere der Kornea 14 und der Sklera 16, für die vorliegende Anwendung. Das von der Kamera 34 aufgenommene Bild des auf dem Kalibrierkörper befindlichen Streifenmusters 32 kann dann auf die tatsächliche Größe und den tatsächlichen Abstand des Streifenmusters 32 von der Kamera 34 kalibriert werden. Für den korrekten Ablauf der Laserbehandlung muss die Positionierung des Ablationslasers überprüft werden. Hierzu wird der Laserscanner 38 kalibriert, beispielsweise indem ein 10 mm mal 10 mm großes Quadrat auf einem geeigneten Material geschossen wird. Die tatsächliche Länge und die tatsächliche Breite der geometrischen Figur werden beispielsweise in die Steuereinrichtung 42 eingetragen und ein geeigneter Korrekturwert errechnet. Eine möglicherweise auftretende Verzerrung wird dabei nicht berücksichtigt.

Zur Ausrichtung der genauen Position der Laserbearbeitungsachse A3 relativ zu der Kamera 34 wird vor der Behandlung mit dem Ablationslaser ein Photopapier an der Position des Kalibrierkörpers bzw. des Auges 12 beschossen. Die Position der auf dem Photopapier erzeugten Strukturen kann mit der Position der Strukturen in dem Bild der Kamera 34 abgeglichen und gegebenenfalls ein Korrekturwert ermittelt werden.

Mit Durchführung der genannten Kalibriervorgänge ist die relative Position von Kamera 34 und Projektor 30 bekannt. Insbesondere ist mit der Aufnahme des Kalibrierkörpers mit der Kamera 34 ein Bild vorhanden, dessen sichtbare Strukturen insbesondere bezüglich deren Abmessungen und Orientierung exakt bekannt sind.

Neben der so durchgeführten Kalibrierung des Gesamtsystems 10 muss von Diagnosesystemen die Tiefe der vorderen Augenkammer 18 gemessen und dem System 10 als Parameter zugeführt werden. Die durchschnittliche Größe für die Vorderkammertiefe beträgt 3,0 mm. Diese Größe stellt einen Bezug zwischen der Iris 20 und der Kornea 14 dar.

Zur Vorbereitung der Behandlung werden der Kopf des Patienten und insbesondere das zu behandelnde Auge 12 innerhalb der Vorrichtung 10 eingerichtet. Zunächst wird der Patient gebeten, einen über die optische Achse der Laserbearbeitungsachse A3 eingekoppelten Fixationslaser zu fixieren. Dies stellt sicher, dass sich das Auge 12 unter möglichst kleiner Winkelabweichung von der idealen Ausrichtung entlang der optischen Achse A3 ausrichtet. Mittels der über die Projektionsachsen A1 und A2 eingestrahlten Linie bzw. Fadenkreuz wird es dem behandelnden Arzt ermöglicht, durch Überlappen der beiden Merkmale auf der Kornea 14 eine möglichst gute laterale Positionierung des Auges 12 zu der optischen Achse A3 zu erreichen. Des Weiteren kontrolliert der behandelnde Arzt visuell, ob sich die Pupille 22 bzw. der Augapfel 13 möglichst mittig zum Kopf des Patienten befinden.

Mit Beginn der Behandlung wird mittels der Kamera 34 ein Referenzbild aufgezeichnet, das zur Ermittlung der Zyklotorsion und einer lateralen Position des Auges 12 zu der optischen Achse A3 dient. Dabei wird ein Bild der Iris 20 ohne das projizierte Streifenmuster 32 aufgenommen.

Im weiteren Verlauf der Behandlung ermittelt die Steuereinrichtung 42 aus dem auf die Iris 20 projizierten Streifenmuster 32 einen $Roll_x$-, einen $Roll_y$- und einen Z-Wert und gibt entsprechende Korrekturwerte für den Laserscanner 38 aus. Dies kann auch schon für den ersten Laserschuss erfolgen. Zur Ermittlung der Zyklotorsion und einer lateralen Position des Auges hinsichtlich der Laserbearbeitungsachse A3 wird ein Kamerabild ohne projizierte Streifen aufgenommen. Sollten die ermittelten X-, Y- oder Z-Werte außerhalb eines zulässigen Wertebereichs liegen, wird die Behandlung unterbrochen. Die Auswertung der genannten beiden Bilder wird im Folgenden detailliert erörtert.

Figur 3 zeigt ein von der Kamera 34 der Vorrichtung 10 aufgenommenes Bild mit dem projizierten Streifenmuster 32 auf der Oberfläche der Iris 20.

Aus der Position der Mitte der Pupille 22 im Kamerabild kann nicht eindeutig ermittelt werden, ob die Position der Pupille 22 durch eine Translation des Augapfels 13 oder durch eine Drehung, auch Rollbewegung genannt, des Augapfels 13 bedingt ist. So kann beispielsweise ein Rollwinkel $Roll_y$ von 5,7° der Iris 20 bei einer Vorderkammertiefe z1 von 3,0 mm einem lateralen Versatz dx von 0,3 mm der Kornea 14 bezüglich der Pupille 22 entsprechen:

$$\tan\left(Roll_y\right) = \tan(5{,}7°) = 0{,}1$$

$$dx = z1 \tan\left(Roll_y\right) = 0{,}3 \text{ mm}$$

Diese vereinfachte Betrachtung berücksichtigt keine Bildverzerrungen durch die Krümmung oder eine Brechungsindexdifferenz der Kornea 14. Da für eine positionsgenaue Laserbehandlung die Lateralposition der Kornea 14 auf 0,05 mm genau bekannt sein soll, ist eine Nachführung des Ablationslaserstrahls durch den Laserscanner 38 während der Behandlung unabdingbar. Während einer solchen Behandlung bewegt sich Kopf eines Patienten lateral unwillkürlich um ±5 mm. Der Augapfel 13 verändert seine Rollwinkelausrichtung auch bei einem bewusst starr gehaltenen Blick um ±3° aufgrund unwillkürlicher Augenbewegungen und kurzer Sakkaden.

Neben der Ermittlung der Lateralposition der Kornea 14 muss auch die exakte Z-Position der Kornea 14 bestimmt werden. Die Z-Position kann nicht mit hinreichender Genauigkeit allein aus dem Durchmesser der Iris 20 oder einem Maßstab von Strukturen, die auf die Iris 20 erkennbar sind, ermittelt werden. Für eine positionsgenaue Laserbehandlung sollte die Z-Position der Kornea 14 auf ±0,25 mm genau bekannt sein, da der die Ablation vornehmende Laserstrahl auf die Oberfläche der Kornea 14 fokussiert wird und eine falsche Fokustiefe nachteilige Auswirkungen auf das Behandlungsergebnis hat. Zur Minimierung einer Bewegung des Auges 12 in Z-Richtung wird der Kopf des Patienten auf einer harten Unterlage aufgesetzt. Weitere Effekte, die eine Bewegung der Kornea 14 in Z-Richtung zur Folge haben, aber vernachlässigt werden, sind ein synchron mit dem Herzschlag um ca. ±0,03 mm veränderlicher Augapfeldurchmesser sowie Muskelbewegungen, die in Abhängigkeit von dem Fokussierzustand der Augenlinse und dem Öffnungszustand der Pupille 22 die Kornea 14 deformieren. Während der Behandlung wird ein Verbleib der Z-Position der Kornea 14 innerhalb eines Toleranzfensters überwacht. Bei einem Verlassen des Toleranzfensters wird die Behandlung rochen und es muss eine erneute Fokussierprozedur durchgeführt werden.

Zunächst wird die Ermittlung der Winkelkoordinaten $Roll_x$ und $Roll_y$ sowie der Z-Koordinate beschrieben:

Die Ermittlung der $Roll_x$-, $Roll_y$- und der Z-Positionswerte erfolgt anhand eines Bildes der Kamera 34, bei dem zumindest die Iris 20 das projizierte Streifenmuster 32 zeigt. Dabei wird von der Steuerungseinrichtung 42 des Systems 10 jedes N-te, beispielsweise jedes zweiunddreißigste, Bild der Kamera 34 ausgewertet. In diesem aufgenommenen ersten Bild wird für jeden Streifen des Streifenmusters 32 individuell eine Geradengleichung aufgestellt. Jeder einzelne Streifen ist individualisiert erfassbar. Dies kann beispielsweise dadurch geschehen, dass ein zentraler Streifen gewissermaßen markiert wird, beispielsweise durch eine größere Breite - wie dies beispielsweise in Figur 3 dargestellt ist - oder beispielsweise durch eine Strukturierung eines ausgewählten Streifens.

Vor einem Ermitteln der Geraden in dem ersten Bild kann das Bild vorverarbeitet werden. Dazu wird das Kamerabild durch Filteroperationen so verbessert, dass sich Linien klar vor dem Hintergrund abheben. Dazu wird in einer Bildrichtung, beispielsweise in einer horizontalen Bildrichtung, eine Korrelation mit einer Linienvorlage, auch Linientemplate genannt, durchgeführt. Das Linientemplate zeigt synthetische, also im Bild erzeugte, Linien im Bereich der Iris. Der Verlauf der linienförmigen Strukturen kann sich beispielsweise aus einem Bild des Kalibrierkörpers ergeben. Das Linientemplate stellt also ein vorher ermitteltes - beispielsweise aus Berechnungen generiertes oder an einem Kalibrierkörper aufgenommenes - Bild dar, das mögliche auftretende Linien wiedergibt. Bei abweichenden Z-, $Roll_x$- und/oder $Roll_y$-Parametern kann aus einem Geometriemodell der Abbildungsoptiken wie beispielsweise den Gegenstands- und Bildweiten sowie dem Triangulationswinkel der Verlauf der linienförmigen Strukturen bestimmt werden. Das Template kann beispielsweise in einer vorangehenden Kalibrierung für einen bestimmten Projektor bestimmt werden. Eine Korrelation mit dem Linientemplate verstärkt in jeder Zeile Pixel, die aufgrund ihrer eigenen Intensität und der ihrer Nachbarn zu einer Linie gehören könnten. Gleichzeitig werden ein möglicherweise vorhandenes thermisches Rauschen des Kamerasensors oder störende Strukturen in der Iris durch diese Korrelationsfilterung abgeschwächt. Eine vergleichbare Filteroperation wird in einer weiteren Bildrichtung, beispielsweise in der vertikalen Richtung, durchgeführt. Auch hier werden Pixel hervorgehoben, die zu einem gleichförmigen Muster wie etwa einer Linie gehören könnten.

In einem weiteren Schritt kann eine sogenannte Skelettierung durchgeführt werden, bei der beispielsweise die äußeren Pixel einer Linie Schritt für Schritt entfernt werden, um eine Linie mit der Breite eines Pixels zu erhalten. Nach diesen Vorverarbeitungen zeichnen sich die linienförmigen Strukturen, also beispielsweise Geraden oder Kreise, als ein Pixel breite Striche im Bild ab.

Zur Ermittlung der Geradengleichungen werden zwei alternative Verfahren angewandt. Zur Ermittlung von Parametern, welche die linienförmigen Strukturen mathematisch wiedergeben, wird als erste mögliche Alternative eine Houghtransformation auf das Bild angewandt. Dabei wird das gegebenenfalls vorverarbeitete Kamerabild in einen Raum transformiert, der jeden Pixel bezüglich seiner Zugehörigkeit zu einer linienförmigen Struktur bewertet. Bei der Houghtransformation werden geradenförmige Linienobjekte in dem zweidimensionalen Kamerabild durch einen Abstandsvektor r zum Bildmittelpunkt und eine Geradensteigung, beispielsweise ein Winkel zur X-Achse, charakterisiert und entsprechend ihrer Gewichtung als Grauwert auf einen Pixel mit den Koordinaten (r, a) im zweidimensionalen Houghraum übertragen. Ähnliches gilt selbstverständlich auch für kreisförmige Objekte, bei einem eventuell elliptisch verzerrten Kreis können es beispielsweise die Koordinaten des Mittelpunkts sowie die Längen von Ellipsenachsen sein. Jeder Pixel des vorverarbeiteten Kamerabildes wird hinsichtlich seiner Zugehörigkeit zu einer linienförmigen Struktur überprüft und bildet in dem Houghraum einen Punkt, dessen Koordinaten von seiner Zugehörigkeit zu einer linienförmigen Struktur abhängen. Aufgrund der Pixelstruktur des vorverarbeiteten Kamerabildes ergibt sich in dem Houghraum eine entsprechende Matrixstruktur, auch Voting-Matrix genannt. In dieser Matrix sind die linienförmigen Strukturen anhand einer Häufung von Punkten in der Matrix erkennbar.

Es kann bei der Bewertung der ermittelten Strukturen eine Gewichtung der einzelnen linienförmigen Strukturen oder Teilen derselben vorgenommen werden. Es können beispielsweise Streifen am äußeren Rand der Iris 20 - nahe an der Grenze zur Sklera 16 - besonders stark gewichtet werden. Da die Iris 20 vom Vorderkammerwasser umgeben ist und kein starres Gebilde ist, kann die Z-Position der Iris 20 am Pupillenrand 22 schwanken -je nach Linsen-Akkommodation und Pupillenöffnung während der Behandlung kann eine Schwankung um bis zu 1 mm entstehen. Es hat sich gezeigt, dass die Iris im Bereich ihres äußeren Umfangs - nahe am Aufhängepunkt - eine geringere Variation aufweist.

Neben den projizierten linienförmigen Strukturen auf der Iris 20 können auch solche auf der Sklera 16 nahe am Limbus 15, der Grenze zwischen weißer Sklera 16 und farbiger Iris 20, ausgewertet werden. Ferner besteht die Möglichkeit, aus dem Umriss des Limbus 15 - gegebenenfalls ohne projizierte Strukturen - auf dessen Verkippung zu schließen: Aus einer Kreisform des Limbus 15 wird bei einer Rotation des Augapfels 13 eine elliptische Form. In der bevorzugten Anordnung ist die Kamera jedoch frontal auf das Auge 12 gerichtet, so dass sich die Breite des Limbus 15 proportional zum Kosinus des Rollwinkels nur geringfügig ändert. Mittel einer schrägstehenden Kamera kann die Empfindlichkeit dieser Methode in einer Dimension verbessert werden. Es wären dann vorteilhafterweise zwei Kameras, die aus x- und y-Richtung schräg auf den Augapfel 13 ausgerichtet sind, vorzusehen.

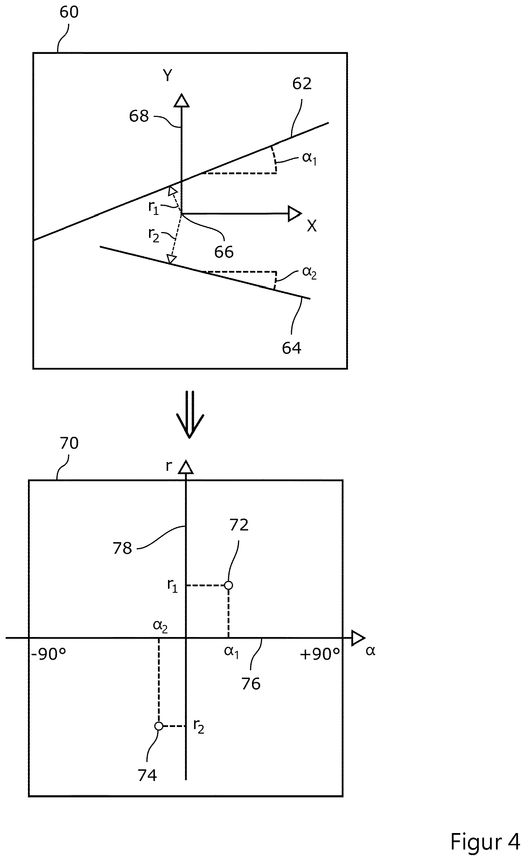

Figur 4 veranschaulicht das Prinzip einer Houghtransformation. Ein erstes Diagramm 60 zeigt zwei Geradenabschnitte 62, 64 in einem kartesisches Koordinatensystem. Die Geraden 62, 64 sind gemäß der Hesseschen Normalform parametrisiert, die einen Abstandsvektor r von dem Ursprung 66 sowie einen Winkel a, hier beispielsweise mit der waagrecht liegenden X-Achse 66, die senkrecht zu der im Bild vertikal verlaufenden y-Achse 68 verläuft, einschließt. Die erste Gerade 62 wird durch den Parametersatz $(r_1, \alpha_1)$ charakterisiert, die zweite Gerade durch den Parametersatz $(r_2, \alpha_2)$. Mittels der Houghtransformation werden die Geraden 62, 64 in den Houghraum transformiert und dort als erster Punkte $(r_1, \alpha_1)$ 72 und als zweiter Punkt $(r_2, \alpha_2)$ 74 dargestellt. In dem Diagramm 70 der Figur 4 ist der Houghraum dargestellt. An der Abszisse 76 ist der Winkel $\alpha$ angetragen, den eine Gerade mit der X-Achse 66 im kartesischen Koordinatensystemen 60 einschließt. Die Ordinate 78 trägt die mit einem Vorzeichen behaftete Abstandsinformation r. Je mehr Pixel aus dem kartesischen Koordinatensystem 60 einer Gerade in dem Houghraum zugeordnet werden und damit als Punkt in dem Diagramm 70 eingetragen werden, umso höher wird der Graustufenwert des Punkts an der entsprechenden Stelle im Houghdiagramm 70.

Die so ermittelten Parameter der linienförmigen Strukturen, also beispielsweise Aufpunkte und Steigungswinkel bei Geraden, werden nun mit dem tatsächlich projizierten Streifenmuster 32 korreliert. Dies wird mithilfe eines Vorlagenabgleichs im Houghraum durchgeführt. Dazu werden alle Streifenmuster, deren Positions- und Lageparameter plausibel erscheinen, als Vorlagen, auch Templates genannt, ermittelt und in den Houghraum transformiert. Diese transformierten Vorlagen werden nun mit dem tatsächlich ermittelten, in den Houghraum transformierten, Kamerabild korreliert. Hohe Korrelationswerte ergeben sich an Stellen, an denen die Houghraum-Vorlage eine linienförmige Struktur aufweist, die auch in dem in den Houghraum transformierten Kamerabild vorhanden sind. Die Vorlage mit dem höchsten Korrelationswert wird als das von der Kamera 34 aufgenommene Streifenmuster 32 des Projektors 34 auf der Iris 20 angesehen. Aus der so ermittelten Vorlage sind eine Position aller linienförmigen Strukturen im Bild, die Abstände der linienförmigen Strukturen voneinander und gegebenenfalls Winkel- oder Verzerrungswerte zu entnehmen. Die für den Vorlagenabgleich verwendeten Vorlagen können beispielsweise ein Satz computergenerierter houghtransformierter Streifen-Vorlagen seien.

Zur Verbesserung der Genauigkeit der Positionsparameter der linienförmigen Strukturen in dem Kamerabild kann eine Rückprojektion der parametrisierten linienförmigen Strukturen in das Kamerabild vorgenommen werden. Hierzu wird das ermittelte parametrisierte linienförmige Muster in Subpixel-Schritten innerhalb des Kamerabilds verschoben und/oder verzerrt und anschließend das entstandene Bild mit dem ursprünglichen Kamerabild korreliert. Bei diesem die Genauigkeit der Position der linienförmigen Strukturen im Bild verbessernden Verfahren kann ein Optimierungsverfahren, wie beispielsweise das Gradientenverfahren, zum Einsatz kommen, da der Suchraum in dem Bild ohne Pixel-Rasterung und damit entsprechend groß ist.

Aus den so ermittelten parametrisierten linienförmigen Objekten kann die Verkippung der als eben angenommenen Iris 20 ermittelt werden: Handelt es sich bei den linienförmigen Objekten um Geraden, kann aus den Abständen der Geraden und aus der Steigung der Geraden der $Roll_x$- und der $Roll_y$-Winkel bestimmt werden. Aus der Position der Geraden in dem Kamerabild kann der Z-Abstand der Ebene der Iris 20 zu der Kamera 34 ermittelt werden.

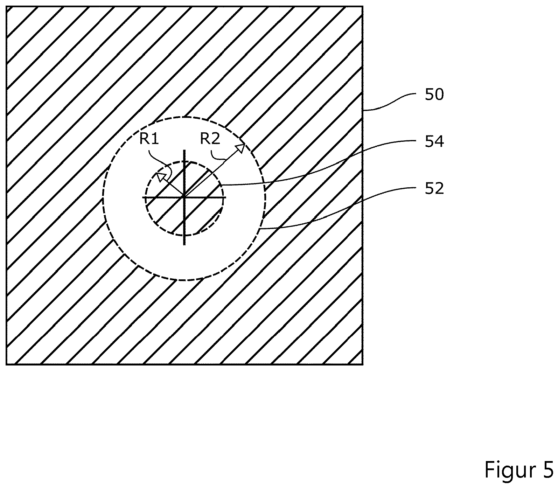

Zur leichteren Ermittlung der in dem Bild der Kamera 34 liegenden linienförmigen Objekte kann eine Maske verwendet werden. Eine solche ist Figur 5 gezeigt. Die Maske blendet alle Bildinhalte um die Iris 20 herum und innerhalb der Pupille 22 aus. Hierzu sind zwei Kreisbögen mit dem Radius R1 für den Durchmesser der Pupille 22 und R2 mit dem äußeren Radius der Iris 20 definiert.

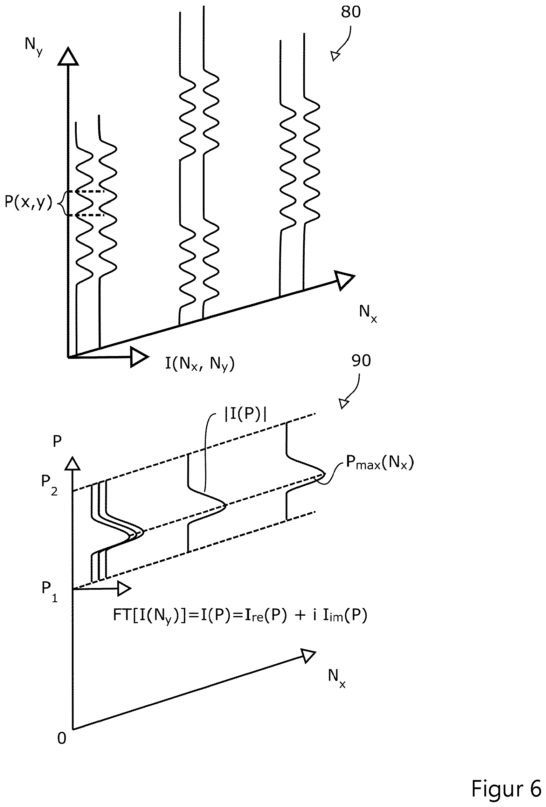

Alternativ zu einer Auswertung des Kamerabilds mittels einer Houghtransformation kann die Auswertung des Streifenmusters 32 mit einer eindimensionalen Fouriertransformation durchgeführt werden. Bevorzugterweise sind die Abstände der Linien des Streifenmusters gleichförmig oder variieren nur gering, um die Qualität der Fouriertransformation hoch zu halten. Dies ist in den Diagrammen 80 und 90 der Figur 6 dargestellt. Diagramm 80 zeigt das von der Kamera 34 aufgenommene Bild der Iris 20 als Intensitätsverteilung $I(N_x, N_y)$ der einzelnen Pixel $(N_x/N_y)$ entlang einer Pixelspalte $N_x$. Die einzelnen Streifen stellen sich als nebeneinanderliegende Intensitätspeaks dar, in der Mitte des Diagramms macht sich die Pupille als fehlende Intensität bemerkbar. Die Verkippung der Iris 20 wird aus dem Verlauf von Periode und Lage der einzelnen Streifen des projizierten Streifenmusters 32 beispielsweise entlang der X-Richtung abgeleitet. Hierzu wird die Pixelintensität $I(N_x, N_y)$ für jede X-Pixelspalte eindimensional fouriertransformiert:

$$I(P, N_x) = \sum_{N_y\ in\ Maske} I(N_x, N_y) \left( \cos\left(\frac{2\pi}{P} N_y\right) + i \sin\left(\frac{2\pi}{P} N_y\right) \right)$$

Auch hier kann die Maske, wie sie in Figur 5 gezeigt ist, eingesetzt werden. Die Fouriertransformation wird nur für einige Periodenwerte P im Wertebereich P1...P2 der Streifenperiode durchgeführt, da schon vorher bekannt ist, bei welchem Periodenwert $P_{max}$ das Maximum des Betrags der Fouriertranformierten $I(P, N_x)$ liegt. Daher genügt es, für einige P-Werte um das Maximum $P_{max}$ herum die Fouriertransformierte zu berechnen. Dies ist in Diagramm 90 dargestellt. Die Streifenperiode liegt im Schwerpunkt des Betrags $|I(P,N_x)|$ der Fouriertransformierten bei $P=P_{max}$ und ermöglicht Aussagen über den Abstand der projizierten Geraden voneinander. Aus dem Mittelwert von $P_{max}$ wird der Winkel Roll$_x$ bestimmt. Ein Rollen des Auges um eine Achse wie beispielsweise die x-Achse führt zu einer Änderung der Streifenperiode Pmax im Kamerabild. Die Phase $\phi$ der komplexwertigen Fouriertransformierten wird in dem Punkt $P=P_{max}$ bestimmt:

$$\phi(N_x) = \arctan\left(\frac{Im(I)}{Re(I)}\right); I(P)\ bei\ P = P_{max}$$

Die Phase $\phi$ gibt Aufschluss über die Y-Position des Streifenmusters 32 im Kamerabild. Aus der mittleren Y-Position des Streifenmusters 32 kann die Z-Position der Iris 20 bestimmt werden. Der zweite Winkelparameter Roll$_y$ kann aus der Steigung, d.h. dem Verlauf der Phase in X-Richtung, ermittelt werden.

Mit den beschriebenen beiden Verfahren - der Houghtransformation und der Fouriertransformation - können die Verkippung der Iris bezüglich der Winkel Roll$_x$ und Roll$_y$ sowie der Versatz der Iris entlang der Z-Achse A3 ermittelt werden. Die noch fehlenden Größen eines lateralen Versatzes der Iris 20 sowie der Zyklotorsion - Roll$_z$ - werden anhand eines Kamerabilds ohne projiziertes Streifenmuster 32 ermittelt. Hierbei wird das Kamerabild ohne projizierte Streifen mit einem Referenzbild verglichen und anhand von Merkmalen der Iris 20 eine Zyklotorsion, also eine Verdrehung um den Winkel Roll$_z$, ermittelt. Des Weiteren wird der Rand der Pupille 22 bestimmt, um so einen Durchmesser der Pupille 22 und deren Mittelpunkt zu erhalten. Es kann zusätzlich oder alternativ der Übergang der Iris 20 zur weißen Sklera 16, also der Limbus 15, ermittelt werden, um auch hier einen Durchmesser und einen Mittelpunkt zu erhalten.

Bei der Aufnahme der Kamerabilder mittels der Kamera 34 können zwei verschiedene Aufnahmemodi verwendet werden. Die in dem vorliegenden Ausführungsbeispiel verwendete Kamera 34 kann beispielsweise eine CMOS-Kamera sein und Bilder mit einer Frequenz beispielsweise von über 1000 Hz, etwa 1200 Hz, aufnehmen. Für die Aufnahme der Iris mit dem projizierten Streifenmuster 34 wird eine bestimmte Auflösung, etwa 512 mal 512 Pixel verwendet, und nur jedes N-te, beispielsweise jedes zweiunddreißigste, Bild ausgewertet. Für die Aufnahme des Kamerabilds ohne projizierte Streifen kann beispielsweise nur die halbe Auflösung, etwa 256 mal 256 Pixel, verwendet werden. Hierzu kann beispielsweise nur jede zweite Zeile der Kamera 34 ausgelesen werden. Zusätzlich können zwei Pixel einer Zeile zu einem Pixel zusammengefasst werden. Auf diese Weise wird nur ein Viertel aller zur Verfügung stehenden Pixel erfasst, was die Auswertung deutlich beschleunigt.

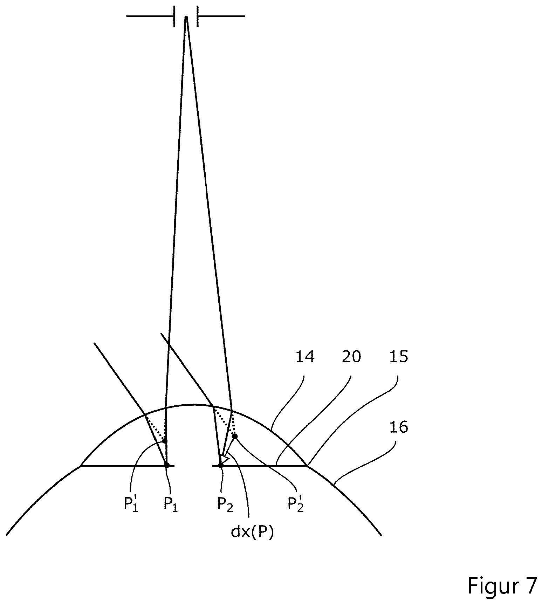

Die Position der Kornea 14 als Koordinaten X, Y bestimmt sich aus dem Nullpunkt der Iris 20 und einer Parallaxe der Position der Kornea 14 aufgrund der Rotation des Augapfels 13 aus der Nullstellung. Diese Parallaxe berechnet sich aus den Rollwinkeln Roll$_x$, Roll$_y$ und der Vorderkammertiefe. Dies ist in Figur 7 dargestellt. Im einfachsten Fall wird die Lichtablenkung durch die gekrümmte Kornea 14 vernachlässigt und es ergeben sich die Punkte P$_1$', P$_2$'. Alternativ kann eine Methode zur Korrektur der daraus resultierenden systematischen Fehler der Position mit einem ortsabhängigen 3D-Verschiebungsvektor dX(P) angewandt werden, der in Figur 7 beispielhaft für den Punkt P$_2$ gezeigt ist. Zur Ermittlung des Verschiebungsvektors dx(P) kann entweder ein computersimuliertes Raytracing des optischen Systems oder eine Kalibrierung der Streifenprojektion mit dem Kamerasystem eingesetzt werden:

Bei einem computersimulierten Raytracing des optischen Systems kann ein Strahl beispielsweise eines des projizierten Streifens bis zur Iris 20 gerechnet werden. Die Optik bilden die Cornea und das Vorderkammerwasser eines durchschnittlichen Auges. Die Iris 20 bildet eine lichtstreuende Planfläche. Von der Iris 20 ausgehend kann weiter ein Strahl bis zur Blendenöffnung der Kamera 34 gerechnet werden. Aus diesen Berechnungen kann dX(P) ermittelt und als Korrekturwert für die (X, Y, Z)-Position verwendet werden. In diesem Falle kann die Projektion mit einem frei stehenden Kalibrierkörper kalibriert werden.

Alternativ kann bei der oben beschrieben Kalibrierung des Projektions-/Kamerasystems beispielweise auf den Kalibrierkörper eine Plankonvexlinse aufgesetzt werden. Die Plankonvexlinse kann so ausgestaltet sein, dass sie die optischen Eigenschaften eines durchschnittlichen Auges wie Krümmungsradius, Brechungsindex und/oder Abstand zur Iris möglichst gut nachbildet. Damit kann der Verschiebungsvektor dx(P) bereits in der Kalibrierung ermittelt werden und in der Kalibrierkennlinie enthalten sein.

Insgesamt werden aus den zwei aufgenommenen Kamerabildern mittels der oben beschriebenen Auswerteverfahren folgende Daten gewonnen:

Die Orientierung der Kornea 14 ergibt sich aus den Rollwinkeln $Roll_x$ und $Roll_y$ der Ebene der Iris 20, wie sie aus dem ersten Kamerabild mit dem projizierten Streifenmuster 32 ermittelt wurden. Aus diesem ersten Kamerabild wird auch die Z-Koordinate der Kornea 14 bestimmt. Der Winkel $Roll_z$, also die Zyklotorsion, wird aus dem zweiten Kamerabild ohne projiziertes Streifenmuster 32 bestimmt. Aus diesem zweiten Kamerabild ergibt sich auch die Position der Kornea 14 als kartesische Koordinaten X, Y, korrigiert mittels der Rollwinkel $Roll_x$, $Roll_y$ wie beschrieben.

**Patentansprüche**

1. Verfahren zur Bestimmung von Größen für die Position und die Lage der Kornea eines Auges, mit den Schritten:

   Erzeugen linienförmiger Strukturen auf der Iris des Auges mittels eines Projektors (30);
   Aufnehmen eines ersten Bildes des Auges mit den linienförmigen Strukturen mittels einer Kamera; **dadurch gekennzeichnet, dass** das Verfahren den folgenden Schritt umfasst:
   Bestimmen durch eine Steuereinrichtung (42), mittels einer Korrelation der linienförmigen Strukturen auf der Iris des Auges des ersten Bildes mit einer Linienvorlage, eines Abstands und von Parametern, welche die Lage der linienförmigen Strukturen relativ zu der Kamera charakterisieren, wobei die Iris als eben angenommen wird.

2. Verfahren nach Anspruch 1, mit dem Schritt des Bestimmens des Abstands und die Lage der Kornea charakterisierenden Parametern mittels des Abstands und die Lage der linienförmigen Strukturen charakterisierenden Parametern.

3. Verfahren nach Anspruch 1 oder2, wobei das Bestimmen von die Lage charakterisierenden Parametern das Bestimmen eines ersten Verkippungswinkels und eines zweiten Verkippungswinkels der linienförmigen Strukturen, insbesondere um Achsen senkrecht zu einer Kameraachse, umfasst.

4. Verfahren nach einem der Ansprüche 1 bis 3, wobei das Bestimmen des Abstands und von die Lage charakterisierenden Parametern ein Ermitteln von die linienförmigen Strukturen charakterisierenden Parametern, insbesondere die Parameter Position und den Winkel der linienförmigen Strukturen, insbesondere die Parameter Z sowie Rollx und Rolly der linienförmigen Strukturen, umfasst.

5. Verfahren nach Anspruch 4, wobei das Bestimmen des Abstands und von die Lage charakterisierenden Parametern ein Anwenden einer Houghtransformation umfasst.

6. Verfahren nach Anspruch 3 oder 4, wobei das Bestimmen des Abstands und von die Lage charakterisierenden Parametern ein Anwenden einer eindimensionalen Fourier-Transformation umfasst und insbesondere die charakterisierenden Parameter eine Periode entlang einer Richtung sowie eine Phase umfassen.

7. Verfahren nach einem der vorhergehenden Ansprüche, wobei das Bestimmen des Abstands und von die Lage charakterisierenden Parametern das Bestimmen, in dem ersten Bild, einer Position der Gesamtheit der linienförmigen Strukturen, eines Abstands der linienförmigen Strukturen voneinander und/oder eines Winkels der Gesamtheit der linienförmigen Strukturen umfasst.

8. Verfahren nach einem der Ansprüche 3 bis 7, wobei das Bestimmen des Abstands und von die Lage charakterisierenden Parametern ein Einfügen geometrischer Figuren anhand der ermittelten Parameter in das erste Bild umfasst, wobei vor dem Einfügen die ermittelten Parameter verändert werden, insbesondere in einem Subpixel-Maßstab.

9. Verfahren nach einem der vorhergehenden Ansprüche, mit den Schritten:

   Aufnehmen eines zweiten Bildes des Auges ohne linienförmige Strukturen mittels einer Kamera;
   Bestimmen, mittels des zweiten Bildes, einer lateralen Position und/oder einer Zyklotorsion des Auges relativ zu der Kamera.

10. Verfahren nach Anspruch 9, wobei der Schritt des Bestimmens der Zyklotorsion und/oder der lateralen Position des Auges ein Aufnehmen eines Referenzbildes umfasst

11. Verfahren nach einem der vorhergehenden Ansprüche, mit dem Schritt:
    Ausgeben von die Position oder/und die Lage der Kornea des Auges charakterisierenden Parametern, insbesondere der Z-Position sowie der Rollx- und Rolly-Werte.

12. Verfahren nach einem der vorhergehenden Ansprüche, wobei das Bestimmen des Abstands und von die Lage charakterisierenden Parametern ein Gewichten der linienförmigen Strukturen am äußeren Rand der Iris an der Grenzen zur Sklera umfasst.

13. Vorrichtung (10) zur Bestimmung von Größen für die Position und die Lage der Kornea (14) eines Auges (12), mit

    einem Projektor (30) zur Erzeugung linienförmiger Strukturen (32) auf der Iris (20) des Auges (12),
    einer Kamera (34), die mit ihrer Kameraachse auf das Auge ausgerichtet ist, sowie
    einer mit dem Projektor (30) und der Kamera (34) verbundenen Steuereinrichtung (42), die dazu eingerichtet ist, mittels der Kamera (34) ein erstes Bild des Auges (12) mit den linienförmigen Strukturen (32) aufzunehmen, **dadurch gekennzeichnet, dass** die Steuereinrichtung (42) dazu eingerichtet ist,
    anhand einer Korrelation der linienförmigen Strukturen (32) auf der Iris (20) des Auges (12) des ersten Bildes mit einer Linienvorlage einen Abstand und die Lage der linienförmigen Strukturen (32) relativ zu der Kamera (34) charakterisierende Parameter zu bestimmen, wobei die Iris (20) als eben angenommen wird

14. Vorrichtung nach Anspruch 13, wobei die Vorrichtung dazu eingerichtet ist, mittels der Kamera ein zweites Bild des Auges (12) ohne linienförmige Strukturen aufzunehmen, und
    anhand des zweiten Bildes eine Zyklotorsion des Auges (12) und/oder eine laterale Position des Auges, insbesondere des Limbus, zu bestimmen.

15. Vorrichtung nach Anspruch 13 oder 14, wobei die Steuereinrichtung (42) dazu eingerichtet ist, ein Verfahren nach einem der Ansprüche 1-12 durchzuführen.

16. Vorrichtung nach einem der Ansprüche 13 bis 15, wobei die Steuereinrichtung (42) dazu eingerichtet ist, für die Aufnahme des zweiten Bildes nur jede N-te Zeile der Kamera (34) auszulesen oder/und N Pixel zu einem Pixel zusammenzufassen.

17. Vorrichtung nach einem der Ansprüche 13 bis 16, wobei eine Beleuchtungseinrichtung (40) vorgesehen ist, die dazu eingerichtet ist, für die Aufnahme des zweiten Bildes die Kornea (12) zumindest teilweise zu beleuchten.


**Claims**

1. A method for determining variables for the position and the orientation of the cornea of an eye, comprising the following steps:

   generating linear structures on the iris of the eye by means of a projector (30);
   recording a first image of the eye with the linear structures by means of a camera;
   **characterized in** the method comprising the following steps:
   determining with a control device (42), by means of a correlation of the linear structures on the iris of the eye of the first image with a line template, a distance and parameters characterizing the orientation of the linear structures relative to the camera, wherein the iris is assumed to be planar.

2. The method as claimed in claim 1, comprising the step of determining the distance and parameters characterizing the orientation of the cornea by means of the distance and parameters characterizing the orientation of the linear structures.

3. The method as claimed in claim 1 or 2, wherein determining parameters characterizing the orientation comprises determining a first tilt angle and a second tilt angle of the linear structures, in particular about axes perpendicular to a camera axis.

4. The method as claimed in any of claims 1 to 3, wherein determining the distance and parameters characterizing the orientation comprises ascertaining parameters characterizing the linear structures, in particular the parameters of position and the angle of the linear structures, in particular the parameters Z, $Roll_x$ and $Roll_y$ of the linear structures.

5. The method as claimed in claim 4, wherein determining the distance and parameters characterizing the orientation comprises applying a Hough transformation.

6. The method as claimed in claim 3 or 4, wherein determining the distance and parameters characterizing the orientation comprises applying a one-dimensional Fourier transformation, and in particular the characterizing parameters comprise a period along a direction and also a phase.

7. The method as claimed in any of the preceding claims, wherein determining the distance and parameters characterizing the orientation comprises determining, in the first image, a position of the totality of the linear structures, a distance between the linear structures and/or an angle of the totality of the linear structures.

8. The method as claimed in any of claims 3 to 7, wherein determining the distance and parameters characterizing the orientation comprises inserting geometric figures into the first image on the basis of the parameters ascertained, wherein before the insertion the parameters ascertained are varied, in particular on a subpixel scale.

9. The method as claimed in any of the preceding claims, comprising the following steps:

   recording a second image of the eye without linear structures by means of a camera;
   determining, by means of the second image, a lateral position and/or a cyclotorsion of the eye relative to the camera.

10. The method as claimed in claim 9, wherein the step of determining the cyclotorsion and/or the lateral position of the eye comprises recording a reference image.

11. The method as claimed in any of the preceding claims, comprising the step of outputting parameters characterizing the position or/and the orientation of the cornea of the eye, in particular the Z-position and also the $Roll_x$ and $Roll_y$ values.

12. The method as claimed in any of the preceding claims, wherein determining the distance and parameters characterizing the orientation comprises weighting the linear structures at the outer edge of the iris at the boundaries with respect to the sclera.

13. An apparatus (10) for determining variables for the position and the orientation of the cornea (14) of an eye (12), comprising

   a projector (30) for generating linear structures (32) on the iris (20) of the eye (12),
   a camera (34), the camera axis of which is aligned with the eye, and also
   a control device (42), which is connected to the projector (30) and the camera (34) and which is configured
   to record a first image of the eye (12) with the linear structures (32) by means of the camera (34), **characterized in** the control device (42) being adapted
   to determine, on the basis of a correlation of the linear structures (32) on the iris (20) of the eye (1") of the first image with a line template, a distance and parameters characterizing the orientation of the linear structures (32) relative to the camera (34), wherein the iris (20) is assumed to be planar.

14. The apparatus as claimed in claim 13, wherein the apparatus is configured

   to record a second image of the eye (12) without linear structures by means of the camera, and
   to determine, on the basis of the second image, a cyclotorsion of the eye (12) and/or a lateral position of the eye, in particular of the limbus.

15. The apparatus as claimed in claim 13 or 14, wherein the control device (42) is configured to carry out a method as claimed in any of claims 1-12.

16. The apparatus as claimed in any of claims 13 to 15, wherein the control device (42) is configured, for the recording of the second image, to read out only every Nth line of the camera (34) or/and to combine N pixels into one pixel.

17. The apparatus as claimed in any of claims 13 to 16, wherein an illumination device (40) is provided, which is configured to at least partly illuminate the cornea (12) for the recording of the second image.


**Revendications**

1. Procédé de détermination de grandeurs concernant la position et l'orientation de la cornée d'un œil, comprenant les étapes suivantes :

   la génération des structures linéaires sur l'iris de l'œil par l'intermédiaire d'un projecteur (30) ;
   la prise d'une première image de l'oeil avec les structures linéaires par l'intermédiaire d'une caméra, **caractérisé en ce que** le procédé comporte l'étape suivante :
   la détermination, au moyen d'un dispositif de commande (42), par l'intermédiaire d'une corrélation des structures linéaires sur l'iris de l'œil de la première image avec un modèle de lignes, d'une distance et des paramètres, qui caractérisent l'orientation des structures linéaires par rapport à la caméra, dans lequel l'iris est pris comme étant plan.

2. Procédé selon la revendication 1, avec l'étape de la détermination de la distance et des paramètres caractérisant l'orientation de la cornée par l'intermédiaire de la distance, et des paramètres caractérisant l'orientation des structures linéaires.

3. Procédé selon la revendication 1 ou 2, dans lequel la détermination des paramètres caractérisant l'orientation comporte la détermination d'un premier angle d'inclinaison et d'un second angle d'inclinaison des structures linéaires, surtout autour des axes perpendiculaires à un axe de la caméra.

4. Procédé selon l'une quelconque des revendications 1 à 3, dans lequel la détermination de la distance et des paramètres caractérisant l'orientation comporte une détermination des paramètres caractérisant les structures linéaires, surtout les paramètres de position et l'angle des structures linéaires, surtout les paramètres Z ainsi que $Roll_x$ et $Roll_y$ des structures linéaires.

5. Procédé selon la revendication 4, dans lequel la détermination de la distance et des paramètres caractérisant l'orientation comporte l'application d'une transformation de Hough.

6. Procédé selon la revendication 3 ou 4, dans lequel la détermination de la distance et des paramètres caractérisant l'orientation comporte une application d'une transformation de Fourier à une dimension, et surtout les paramètres caractérisants comprennent une période le long d'une direction ainsi qu'une phase.

7. Procédé selon l'une quelconque des revendications précédentes, dans lequel la détermination de la distance et des paramètres caractérisant l'orientation comporte la détermination, dans la première image, d'une position de l'ensemble des structures linéaires, d'une distance des structures linéaires les unes des autres et / ou d'un angle de l'ensemble des structures linéaires.

8. Procédé selon l'une quelconque des revendications 3 à 7, dans lequel la détermination de la distance et des paramètres caractérisant l'orientation comporte une insertion des formes géométriques par l'intermédiaire des paramètres déterminés dans la première image, dans lequel, avant l'insertion, les paramètres déterminés sont modifiés, surtout dans une échelle de sous pixels.

9. Procédé selon l'une quelconque des revendications précédentes, avec les étapes suivantes :

   la prise d'une seconde image de l'oeil sans des structures linéaires par l'intermédiaire d'une caméra ;

la détermination, par l'intermédiaire de la seconde image, d'une position latérale et / ou d'une cyclotorsion de l'œil par rapport à la caméra.

10. Procédé selon la revendication 9, dans lequel l'étape de la détermination de la cyclotorsion et / ou de la position latérale de l'œil comporte une prise d'une image de référence.

11. Procédé selon l'une quelconque des revendications précédentes, avec l'étape suivante :
la sortie des paramètres caractérisant la position et / ou l'orientation de la cornée de l'œil, surtout de la position Z ainsi que des valeurs $Roll_x$ et $Roll_y$.

12. Procédé selon l'une quelconque des revendications précédentes, dans lequel la détermination de la distance et des paramètres caractérisant l'orientation comporte une pondération des structures linéaires au bord extérieur de l'iris aux confins à la sclère.

13. Dispositif (10) pour la détermination des grandeurs pour la position et l'orientation de la cornée (14) d'un œil (12) comportant

un projecteur (30) pour la génération des structures linéaires (32) sur l'iris (20) de l'œil (12),
une caméra (34), qui est dirigée vers l'œil avec son axe de la caméra, ainsi que
un dispositif de commande (42) relié au projecteur (30) et à la caméra (34), qui est adapté pour prendre, par l'intermédiaire de la caméra (34), une première image de l'œil (12) avec les structures linéaires (32), **caractérisé en ce que** le dispositif de commande (42) est adapté pour
déterminer, par l'intermédiaire d'une corrélation des structures linéaires (32) sur l'iris (20) de l'œil (12) de la première image avec un modèle de lignes, une distance et des paramètres caractérisant l'orientation des structures linéaires (32) par rapport à la caméra (34), dans lequel l'iris est pris comme étant plan.

14. Dispositif selon la revendication 13, dans lequel le dispositif est adapté pour prendre une seconde image de l'œil (12), par l'intermédiaire de la caméra (34), sans des structures linéaires
et
pour déterminer, par l'intermédiaire de la seconde image, une cyclotorsion de l'œil (12) et / ou une position latérale de l'œil, surtout du limbe.

15. Dispositif selon la revendication 13 ou 14, dans lequel le dispositif de commande (42) est adapté pour réaliser un procédé selon l'une quelconque des revendications 1 à 12.

16. Dispositif selon l'une quelconque des revendications 13 à 15, dans lequel le dispositif de commande (42) est adapté pour ne lire que chaque N-ième ligne de la caméra (34) et / ou regrouper N pixels en un pixel, pour la prise de la seconde image.

17. Dispositif selon l'une quelconque des revendications 13 à 16, dans lequel un appareil d'éclairage (40) est prévu, qui est adapté pour éclairer au moins partiellement la cornée (12) pour la prise de la seconde image.

Figur 1

Figur 2

Figur 3

Figur 4

Figur 5

Figur 6

Figur 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010024407 A1